# FASCICULE DE BREVET EUROPEEN

(11) **EP 2 249 158 B1**
(45) Date de publication et mention de la délivrance du brevet: **16.12.2015**
(21) Numéro de dépôt: 10290206.1
(22) Date de dépôt: 16.04.2010
(51) Int. Cl.: G01N 33/543, B01L 3/00

(54) **Méthode de dosage d'enzymes plasmatiques dans le sang total**
Dosierung von Plasma Enzymen im Blut
Dosage of plasmatic enzymes in blood

(30) Priorité: 20.04.2009 FR 0901894
(43) Date de publication de la demande: 10.11.2010
(73) Titulaire: Commissariat à l'Énergie Atomique et aux Énergies Alternatives, 75015 Paris (FR)
(72) Inventeur: Cubizolles, Myriam-Laure, 38000 Grenoble (FR); Cosnier, Marie-Line, 38000 Grenoble (FR); Faivre, Magali, 38000 Grenoble (FR); Pouteau, Patrick, 38240 Meylan (FR)
(74) Mandataire: Rançon, Xavier Lucien Abel

(56) Documents cités:
- US-A- 3 925 162
- ZHUANG GUI-SHENG ET AL: "Microchip-based capillary electrophoresis for determination of lactate dehydrogenase isoenzymes" JOURNAL OF SEPARATION SCIENCE, vol. 30, no. 9, juin 2007 (2007-06), pages 1350-1356, XP002552607
- HUANG ET AL: "Aspartate Aminotransferase (AST/GOT) and Alanine Aminotransferase (ALT/GPT) Detection Techniques" SENSORS, vol. 6, 31 juillet 2006 (2006-07-31), pages 756-782, XP002552608

## Description

La présente invention concerne une méthode de dosage *in vitro* d'enzymes plasmatiques, telles que les transaminases ou les enzymes NADH dépendantes, dans un échantillon de liquide biologique, par la mesure de la fluorescence du NADH.

Les transaminases sont des enzymes qui catalysent le transfert d'un groupe amine d'un acide aminé à un autre. Chez l'homme, on distingue deux types de transaminases :
- l'ASAT (ASpartate Amino Transférase), AST ou GOT (Glutamate Oxaloacétique Transaminase). Cette enzyme catalyse la réaction (i) suivante : L-aspartate + α-cétoglutarate → oxaloacétate + L-glutamate ;
- l'ALAT (ALanine Amino Transférase), ALT ou GPT (Glutamate Pyruvate Transaminase). Cette enzyme catalyse la réaction (ii) suivante : L-alanine + α-cétoglutarate → pyruvate + L-glutamate.

Les transaminases sont présentes dans tous les tissus, principalement dans le foie et le muscle cardiaque, ainsi que dans les globules rouges. En cas de dommage hépatique (nécrose ou hépatite) ou d'infarctus du myocarde par exemple, les transaminases sont libérées respectivement du foie ou du myocarde vers la circulation sanguine, ce qui entraîne une augmentation de leurs niveaux dans le sang. Le dosage des transaminases dans le sérum ou le plasma des patients constitue un outil de diagnostic des pathologies hépatiques ou cardiaques.

Les principales méthodes de dosage des transaminases plasmatiques sont basées sur des méthodes optiques, électrochimiques, chromatographiques ou radiochimiques (voir pour revue Huang et al., Sensors, 2006, 6:756-782). Les méthodes optiques sont utilisées en routine ; parmi ces méthodes, on distingue :

### - la colorimétrie :

Cette méthode de dosage est réalisée à partir de sérum ou de plasma. Après arrêt des réactions enzymatiques catalysées par l'AST ou l'ALT, on utilise un sel de diazonium qui réagit non seulement avec l'oxaloacétate (un des produits de la réaction catalysée par l'AST) pour donner un composé de couleur rouge, mais aussi avec le L-glutamate (un des produits des réactions catalysées par l'AST et l'ALT) pour donner, en présence de glutamate déshydrogénase, un composé de couleur verte ; il suffit ensuite de mesurer la densité optique (DO) du sérum ou du plasma coloré en rouge ou en vert. La loi de Beer-Lambert permet alors de relier la densité optique d'une solution à sa concentration molaire (c) : DO = εlc, « ε » étant le coefficient d'absorption spécifique du composé coloré et « 1 » l'épaisseur de la cuve contenant la solution (sérum ou plasma). Les inconvénients de cette méthode sont principalement le calibrage des cuves qui doit être effectué pour chaque dosage et la nécessité d'arrêter les réactions enzymatiques pour effectuer le dosage. Il est également possible de mettre en oeuvre cette méthode après immobilisation des réactifs sur phase solide, par exemple sur membrane. Différents dispositifs de dosage des transaminases, basés sur une méthode colorimétrique après immobilisation des réactifs sur phase solide, ont été proposés. Parmi ces dispositifs, on peut citer le dispositif de biologie délocalisée (« *point-of-care* ») dénommé CholestechLDX® (Cholestech). Le dosage des transaminases AST et ALT à l'aide de ce dispositif s'effectue à partir d'un échantillon de 35 µL de sang total capillaire, et comprend les étapes suivantes : le sang est déposé dans le puits d'une cassette (consommable du dispositif) et migre par capillarité dans la cassette qui comporte un filtre retenant spécifiquement les globules rouges ; le plasma résultant est ensuite mis au contact de réactifs séchés sur une membrane, conduisant à une réaction colorimétrique ; le système optique du dispositif mesure alors l'intensité de la couleur finale de la réaction par photométrie de réflectance, la coloration bleue mesurée étant d'une intensité proportionnelle à la concentration en transaminases présentes dans l'échantillon (Demandes Internationales WO 2004/90555 et WO 2005/044429) ;

### - la spectrophotométrie d'absorption UV à 340 nm :

Selon cette méthode, c'est l'activité d'enzymes qui dégradent l'oxaloacétate et le pyruvate, produits respectivement par l'AST et l'ALT, qui est mesurée lors de réactions enzymatiques secondaires. Ces réactions enzymatiques sont catalysées respectivement par les enzymes MDH (Malate DésHydrogénase; EC 1.1.1.37) et LDH (Lactate DésHydrogénase ; EC 1.1.1.27), en présence de NADH (β-nicotinamide adénine dinucléotide) :
- la MDH catalyse la réaction (iii) suivante : oxaloacétate + NADH + H⁺ → malate + NAD⁺ ;
- la LDH catalyse la réaction (iv) suivante : pyruvate + NADH + H⁺ → L-lactate + NAD⁺.

Le NADH (forme réduite) absorbe dans l'ultraviolet (UV) à 340 nm, alors que le NAD⁺ (forme oxydée) n'absorbe pas à cette longueur d'onde. On peut donc mesurer l'activité enzymatique des réactions secondaires catalysées par la LDH ou la MDH en suivant la décroissance de l'absorbance UV à 340 nm (correspondant à la consommation de NADH au cours des réactions enzymatiques secondaires en fonction du temps), et ainsi en déduire les activités enzymatiques de l'AST et de l'ALT. Le dosage des transaminases AST et ALT par cette méthode s'effectue, non pas directement sur du sang total, mais à partir de sérum ou de plasma (Karmen et al., J. Clin. Invest., 1955, 34:131-133 ; Brevet américain US 3,925,162 et Demande de Brevet européen EP 415 188). En effet, le sang total, qui comprend les globules rouges, est un milieu très absorbant, notamment à la longueur d'onde de 340 nm, et des taux variables d'hématocrite (défini comme le rapport, exprimé en pourcentage, entre le volume de globules rouges obtenu en centrifugeant un échantillon de sang total et le volume de cet échantillon) de patients différents présentant par ailleurs un taux de transaminases identique pourrait donner lieu à des résultats de dosage de transaminases différents car la variation d'absorption UV serait fonction de la quantité de globules rouges et non fonction du taux de transaminases. Conformément aux recommandations de l'IFCC (*International Federation of Clinical Chemistry and Laboratory Medicine*), le dosage de l'AST selon cette méthode comprend les étapes suivantes : mélanger le plasma ou le sérum avec du L-aspartate, du NADH, du phosphate de pyridoxal (PPal ou PLP pour pyridoxal-phosphate), une MDH et une LDH dans un tampon approprié ; laisser incuber pendant 5 minutes ; ajouter ensuite l'α-cétoglutarate (de manière à initier la réaction enzymatique (i) ci-dessus ; laisser incuber pendant 90 secondes ; et mesurer la décroissance de l'absorbance UV du NADH pendant 180 secondes (Schumann et al., Clin. Chem. Lab. Med., 2002, 40:725-733). Une phase de latence (ou d'incubation) de 90 secondes est recommandée afin d'obtenir une décroissance linéaire du signal (absorbance UV) mesuré en fonction du temps. Conformément aux recommandations de l'IFCC, le dosage de l'ALT selon cette méthode comprend les mêmes étapes que celles décrites pour le dosage de l'AST, mais il n'est pas utilisé de MDH et le L-aspartate est remplacé par la L-alanine ; l'ajout d'α-cétoglutarate permet d'initier la réaction enzymatique (ii) ci-dessus (Schumann et al., Clin. Chem. Lab. Med., 2002, 40:718-724).

### - la fluorescence :

Cette méthode fait intervenir les mêmes réactions enzymatiques secondaires que celles décrites pour la spectrophotométrie d'absorption UV ci-dessus. Le NADH, excité à une longueur d'onde de 340 nm, fluoresce à une longueur d'onde de 460 nm, ce qui n'est pas le cas de sa forme oxydée, le NAD⁺. L'intensité d'émission est alors dépendante de la concentration en NADH dans le milieu étudié. L'activité enzymatique des transaminases AST et ALT peut donc être suivie par la diminution de la fluorescence émise par le NADH à 460 nm, soit en phase homogène, soit après immobilisation sur un support solide. Pour les même raisons qu'exposées ci-dessus, le dosage des transaminases AST et ALT par cette méthode ne peut s'effectuer que sur du sérum ou du plasma (Brevets américains US 3,925,162 et US 5,612,178 ; Demande Internationale WO 91/013169).

Le dosage des enzymes plasmatiques ayant comme cofacteur le NADH (enzymes NADH-dépendantes), telles que les LDH et les MDH, s'effectue, sur sérum ou plasma, par mesure de leur activité enzymatique par spectrophotométrie d'absorption UV du NADH à 340 nm ou par suivi de la décroissance de la fluorescence à 460 nm du NADH consommé au cours de la réaction enzymatique catalysée par ces enzymes (dans le cas du dosage de la MDH, c'est le NADH consommé au cours de la réaction enzymatique (iii) décrite ci-dessus qui est mesuré et dans le cas du dosage de la LDH, c'est le NADH consommé au cours de la réaction enzymatique (iv) décrite ci-dessus qui est mesuré). Le dosage de ces enzymes est réalisé en mélangeant à l'échantillon de sérum ou plasma, leurs substrats (dont le NADH).

Zhuang GS et al. (2007, J. Sep. Sci., 30:1350-6) décrit un dispositif microfluidique pour séparer par électrophorèse capillaire la LDH. Zhuang GS *et al.* décrit également le dosage de la LDH par mesure de la fluorescence du NADH formé lors de la réduction du NAD⁺ par la LDH en présence de lactate.

Le dosage des LDH, par exemple, est utilisé pour le diagnostic et le suivi d'affections hépatiques et cardiaques, et de certains cancers (poumon et reins).

Les Inventeurs se sont donné pour but de pourvoir à une méthode de dosage d'enzymes plasmatiques, notamment les enzymes NADH-dépendantes et les transaminases, à partir d'un faible volume (quelques microlitres) de sang total humain et sans séparation préalable des globules rouges, le dosage étant réalisé rapidement après le prélèvement de sang (10 à 30 minutes).

De manière surprenante, les Inventeurs ont mis en évidence que le dosage des enzymes catalytiques plasmatiques NADH-dépendantes, telles que les LDH et MDH, par mesure de leur activité enzymatique (ou concentration catalytique), pouvait être effectué directement dans un échantillon de sang total, c'est-à-dire en présence notamment des globules rouges, en mesurant, dans une chambre microfluidique, la décroissance de la fluorescence du NADH consommé au cours de la réaction enzymatique catalysée par ces enzymes. Cette méthode s'est avérée fiable, rapide et robuste. En outre, elle peut être mise en oeuvre à l'aide d'un dispositif de biologie délocalisée.

Cette méthode peut aussi s'appliquer au dosage d'une enzyme catalytique plasmatique E_{C} ayant comme substrats S₁ et S₂, telle que les transaminases, par mesure de la décroissance de la fluorescence du NADH consommé au cours de réactions enzymatiques secondaires qui sont catalysées par une ou plusieurs enzymes catalytiques NADH-dépendantes ayant pour substrat l'un des produits de la réaction enzymatique catalysée par ladite enzyme catalytique plasmatique E_{C}.

En conséquence, la présente invention a pour objet une méthode de dosage, dans un échantillon de liquide biologique, d'une enzyme catalytique plasmatique NADH-dépendante, par mesure de son activité enzymatique, comprenant une étape de mesure de la décroissance de la fluorescence du NADH, laquelle méthode est caractérisée **en ce que** ledit liquide biologique est du sang total et en ce que l'activité enzymatique de ladite enzyme catalytique est mesurée en suivant la décroissance de la fluorescence du NADH dans une chambre microfluidique de 20 µm à 2 mm d'épaisseur.

On entend par enzyme catalytique plasmatique NADH-dépendante (E_{N}), une enzyme catalytique présente dans le sang qui utilise le NADH comme cofacteur.

A titre d'exemples non limitatifs d'enzymes catalytiques plasmatiques NADH-dépendantes (E_{N}), on peut citer la lactate déshydrogénase (LDH), la malate déshydrogénase (MDH) et l'isocitrate déshydrogénase (IDH).

On entend par chambre microfluidique, un dispositif présentant une veine fluidique susceptible de recevoir de très faibles quantités d'échantillon liquide, de l'ordre de la dizaine de microlitres. Les dimensions réduites de ladite chambre, de 20 µm à 2 mm d'épaisseur, de préférence de 20 µm à 100 µm, plus préférentiellement de 30 µm à 50 µm, et particulièrement de 30 µm, permettent de minimiser les mouvements dans l'échantillon liquide et aux globules rouges de sédimenter en quelques minutes selon l'épaisseur de la chambre microfluidique. En effet, une particularité du sang total est le phénomène de sédimentation du fait de la gravité agissant sur les particules les plus denses, la vitesse de sédimentation des globules rouges étant comprise entre 0,3 et 3 µm/s. Il est donc avantageux de choisir une chambre microfluidique de quelques dizaines de µm d'épaisseur de manière à avoir un temps de sédimentation (ou temps de latence) le plus court possible.

N'importe quel matériau transparent aux longueurs d'ondes d'environ 300 à 550 nm, tel que le plastique et le quartz peut être utilisé pour fabriquer la chambre microfluidique.

Selon un autre mode de mise en oeuvre avantageux de l'invention, ladite chambre microfluidique comprend deux parois, de préférence de faible épaisseur (d'une centaine de µm à quelques mm), sensiblement parallèles et séparées par un ou plusieurs moyens d'espacement, tel(s) que la distance entre lesdites deux parois soit comprise entre 20 µm à 2 mm, de préférence entre 20 µm et 100 µm, plus préférentiellement entre 30 µm et 50 µm, et particulièrement de 30 µm. Les moyens d'espacement peuvent être, de manière non limitative, des plots ou des barres continues ou discontinues.

Selon une disposition avantageuse de ce mode de mise en oeuvre de l'invention, ladite chambre microfluidique comprend au moins une paroi constituée d'un matériau réfléchissant la lumière de fluorescence. L'utilisation d'un matériau réfléchissant la lumière de fluorescence permet, en dépit de l'accumulation des globules rouges au fond de la dite chambre microfluidique, d'augmenter le signal de fluorescence mesuré. Ledit matériau réfléchissant peut être constitué d'oxydes ou de nitrures de silicium, tels que SiO₃ et Si₃N₄, ou de silicium (SiO₂) de préférence de silicium.

Selon une autre disposition avantageuse de ce mode de mise en oeuvre de l'invention, ladite chambre microfluidique comprend une paroi sensiblement plane, en verre ou en silicium, telle que par exemple une lame de microscope, et une paroi également sensiblement plane, en quartz ou en plastique, telle que par exemple une lamelle de microscope ; lesdites parois étant espacées de 20 µm à 2 mm, de préférence de 20 µm à 100 µm, plus préférentiellement de 30 µm à 50 µm, et particulièrement de 30 µm, à l'aide de plots (ou espaceurs) par exemple.

L'échantillon de sang total, de préférence de sang humain, peut être prélevé sur anticoagulant, tel que l'EDTA (acide éthylène diamine tétra acétique), le citrate de sodium ou l'héparine, de préférence le citrate de sodium.

Selon un autre mode de mise en oeuvre avantageux de l'invention, le volume de l'échantillon de sang total est compris entre 0,1 µL et 50 µL, de préférence il est de 3 µL.

Ledit échantillon de sang total peut, en outre, être mélangé aux substrats de ladite enzyme catalytique plasmatique NADH-dépendante (dont le NADH) ainsi qu'à un tampon approprié. De tels tampons sont bien connus de l'homme du métier. A titre d'exemples de tampons, on peut citer le Tris(hydroxyméthyl)aminométhane (Tris) et un mélange constitué de Tris et d'azide de sodium (NaN₃).

L'introduction de l'échantillon de sang total dans ladite chambre microfluidique peut être réalisée à l'aide d'une pompe ou d'un piston, ou par migration capillaire, en évitant la lyse des globules rouges (hémolyse). En effet, les globules rouges peuvent renfermer des enzymes identiques auxdites enzymes catalytiques et qui ne doivent pas être dosées (étant donné que l'on souhaite déterminer l'activité catalytique des enzymes plasmatiques).

Selon un autre mode de mise en oeuvre avantageux de l'invention, 20 µM à 250 µM de NADH est ajouté audit échantillon de sang total.

La fluorescence du NADH est mesurée à l'aide d'un fluorimètre ou d'une caméra appropriée, sensible à la fluorescence, à une longueur d'onde comprise entre 420 nm et 550 nm, de préférence à 460 nm, après excitation du NADH à une longueur comprise entre 300 nm et 400 nm.

Selon un mode de mise en oeuvre préféré de l'invention, la mesure de la décroissance de la fluorescence s'effectue après l'écoulement d'un temps de latence défini par le temps de sédimentation des globules rouges dans ladite chambre microfluidique ; ce temps de latence varie donc en fonction de l'épaisseur de ladite chambre microfluidique.

Selon un autre mode de mise en oeuvre avantageux de l'invention, la mesure de la décroissance de la fluorescence du NADH s'effectue à température ambiante ou à une température d'environ 37°C, de préférence à 37°C.

La décroissance de la fluorescence du NADH peut être mesurée pendant 180 secondes après l'écoulement du temps de latence.

La présente invention a également pour objet une méthode de dosage, dans un échantillon de liquide biologique, d'une enzyme catalytique plasmatique (E_{C}) ayant comme substrats S₁ et S₂, par mesure de l'activité enzymatique de ladite enzyme E_{C}, ladite enzyme E_{C} étant une enzyme catalytique plasmatique dont l'un des produits obtenus au cours de la réaction enzymatique catalysée par celle-ci est lui-même un substrat d'au moins une enzyme catalytique NADH-dépendante (E_{N}) ayant comme cofacteur le NADH, ladite méthode, comprenant au moins les étapes suivantes : a) incubation dudit liquide biologique avec ledit substrat S₁, du NADH, une ou plusieurs enzyme(s) catalytique(s) NADH-dépendante(s) (E_{N}) différentes ayant chacune pour substrat l'un des produits de la réaction enzymatique catalysée par E_{C}, et optionnellement du phosphate de pyridoxal ; b) ajout dudit substrat S₂ de manière à initier les réactions enzymatiques catalysées par les enzymes catalytiques E_{C} et EN ; laquelle méthode est caractérisée :
- en ce que ledit liquide biologique est du sang total, tel que défini ci-dessus,
- en ce qu'après l'ajout du substrat S₂ à l'étape b), le mélange obtenu est immédiatement introduit dans une chambre microfluidique de 20 µm à 2 mm d'épaisseur telle que définie ci-dessus, et
- en ce que l'activité enzymatique de ladite enzyme catalytique E_{C} est mesurée en suivant la décroissance de la fluorescence du NADH dans ladite chambre microfluidique, dans les conditions définies ci-dessus.

Selon un mode de mise en oeuvre avantageux de cet aspect de l'invention, ladite chambre microfluidique est de quelques dizaines de µm d'épaisseur. En effet, dans le cas de l'utilisation d'une chambre microfluidique de 30 µm d'épaisseur pour le dosage des transaminases, la distance parcourue par les globules rouges pour une sédimentation complète dans ladite chambre microfluidique atteint environ 27 µm pendant les 90 secondes de la phase de latence, ce qui correspond à la phase de latence recommandée.

Selon un autre mode de mise en oeuvre avantageux de cet aspect de l'invention, la concentration du NADH dans le mélange obtenu à l'étape a) est de 20 µM à 250 µM.

Le temps d'incubation à l'étape a) définie ci-dessus est compris entre 2 et 30 minutes, de préférence il est de 5 minutes.

Ladite enzyme catalytique (E_{C}) définie ci-dessus peut être toute enzyme catalytique plasmatique dont l'un des produits obtenus au cours de la réaction enzymatique catalysée par celle-ci est lui-même un substrat d'au moins une enzyme catalytique (E_{N}) ayant comme cofacteur le NADH. A titre d'exemple d'enzymes catalytiques (E_{C}), on peut citer l'aspartate amino transférase (AST), l'alanine amino transférase (ALT), la pyruvate kinase, la créatine kinase et la glycérol kinase. L'activité enzymatique de ces enzymes peut être mesurée en utilisant une lactate déshydrogénase (LDH) et/ou une malate déshydrogénase (MDH) en tant qu'enzyme catalytique EN.

Les LDH (EC 1.1.1.27) et les MDH (EC 1.1.1.37) sont bien connues de l'homme du métier. Elles peuvent être isolées de n'importe quel mammifère, de préférence le porc, ou bien peuvent être recombinantes ou synthétiques.

Selon un mode de mise en oeuvre particulier de cet aspect de l'invention, ladite enzyme catalytique E_{C} est l'aspartate amino transférase.

Selon ce mode de mise en oeuvre particulier de l'invention, ledit substrat S₁ est le L-aspartate, ledit substrat S₂ est l'α-cétoglutarate, et lesdites enzymes catalytiques EN sont, soit une MDH, soit une MDH et une LDH ; la concentration du NADH dans le mélange obtenu à l'étape a) est de préférence de 20 à 200 µM, plus préférentiellement 90 µM ; et la fluorescence du NADH est de préférence mesurée à 460 nm après excitation du NADH à une longueur d'onde de 318 nm de préférence.

Selon un autre mode de mise en oeuvre particulier de cet aspect de l'invention, ladite enzyme catalytique E_{C} est l'alanine amino transférase.

Selon ce mode de mise en oeuvre particulier de l'invention, ledit substrat S₁ est la L-alanine, ledit substrat S₂ est l'α-cétoglutarate, et ladite enzyme catalytique EN est une LDH ; la concentration du NADH dans le mélange obtenu à l'étape a) est de préférence de 20 à 250 µM, plus préférentiellement 180 µM ; et la fluorescence du NADH est de préférence mesurée à 460 nm après excitation du NADH à une longueur d'onde de 367 nm de préférence.

La concentration en AST ou en ALT dans l'échantillon de sang exprimée en U/L est obtenue en multipliant la valeur de l'activité enzymatique (concentration catalytique) de l'AST ou de l'ALT exprimée en µkat/l par un facteur f égal à 60 (voir les articles de Schumann *et al.*, 2002, cités ci-dessus).

La présente invention a aussi pour objet l'utilisation d'une chambre microfluidique telle que définie ci-dessus pour doser une enzyme catalytique plasmatique (E_{N} ou E_{C}) telle que définie ci-dessus dans un échantillon de sang total.

La présente invention a en outre pour objet un kit ou coffret pour doser une enzyme catalytique plasmatique E_{C} dans un échantillon de sang total, ladite enzyme E_{C} étant une enzyme catalytique plasmatique dont l'un des produits obtenus au cours de la réaction enzymatique catalysée par celle-ci est lui-même un substrat d'au moins une enzyme catalytique NADH-dépendante (E_{N}) ayant comme cofacteur le NADH, et ladite enzyme E_{C} ayant comme substrats S₁ et S₂, ledit substrat S₁ étant le L-aspartate et ledit substrat S₂ étant l'α-cétoglutarate, ou ledit substrat S₁ étant la L-alanine et ledit substrat S₂ étant l'α-cétoglutarate, caractérisé en ce qu'il comprend une chambre microfluidique telle que définie ci-dessus, et les réactifs suivants : les substrats S₁ et S₂, le NADH, au moins une enzyme catalytique NADH-dépendante (E_{N}), et optionnellement du PLP.

Selon un mode de réalisation du kit ou coffret selon l'invention, il comprend une chambre microfluidique telle que décrite ci-dessus, du L-Asp, une MDH, du NADH, de l'a-cétoglutarate et optionnellement une LDH et du PLP, le kit ou coffret étant utile pour mesurer l'activité enzymatique ou doser l'AST dans un échantillon de sang total humain.

Selon un autre mode de réalisation du kit ou coffret selon l'invention, il comprend une chambre microfluidique telle que décrite ci-dessus, de la L-Ala, une LDH, du NADH, de l'a-cétoglutarate et du PLP, le kit ou coffret étant utile pour mesurer l'activité enzymatique ou doser l'ALT dans un échantillon de sang total humain.

Les inventeurs décrivent un kit ou coffret pour doser une enzyme catalytique plasmatique NADH-dépendante, caractérisé en ce qu'il comprend une chambre microfluidique telle que définie ci-dessus.

Outre les dispositions qui précèdent, l'invention comprend encore d'autres dispositions, qui ressortiront de la description qui va suivre, et qui se réfèrent à des exemples de mise en oeuvre de la méthode objet de la présente invention ainsi qu'aux dessins annexés, dans lesquels :

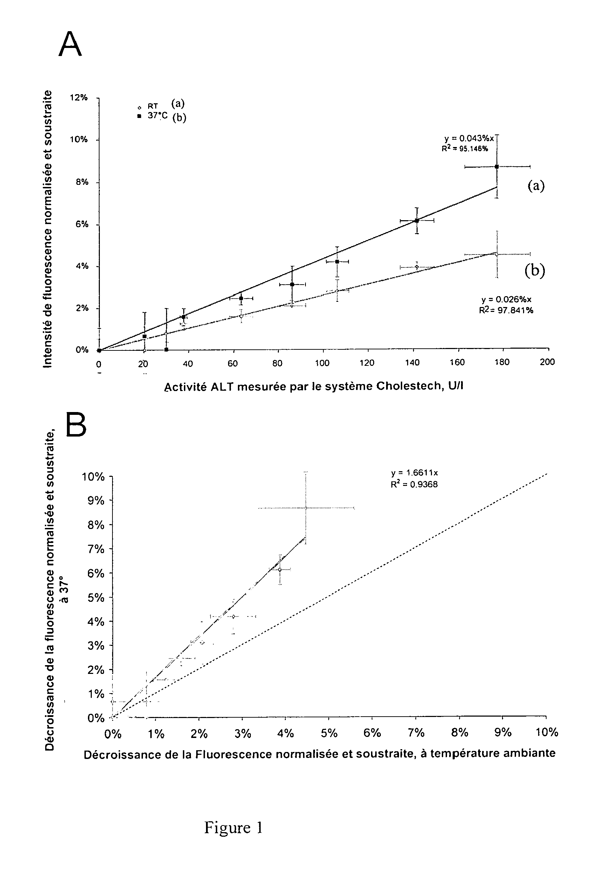

- la Figure 1 représente l'effet de la température sur la mesure de la décroissance de la fluorescence du NADH pour le dosage de l'ALT. A- Corrélation entre la méthode de référence et la méthode selon la présente invention à température ambiante (a) ou à 37°C (b). B- Corrélation entre les mesures selon la méthode de la présente invention à température ambiante et à 37°C ;

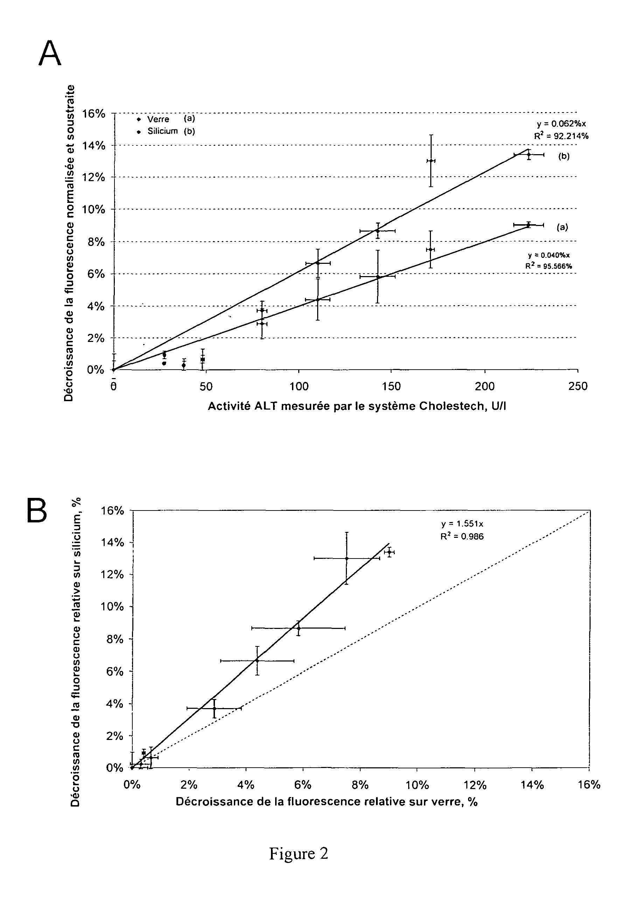

- la Figure 2 représente l'effet du matériau constituant la base de la chambre microfluidique sur la mesure de la décroissance de la fluorescence du NADH pour le dosage de l'ALT. A- Corrélation entre la méthode de référence et la méthode selon la présente invention avec les bases de chambres microfluidiques constituées de verre (a) ou de silicium (b). B- Corrélation entre les mesures effectuées avec des bases de chambres microfluidiques constituées de verre ou de silicium ;

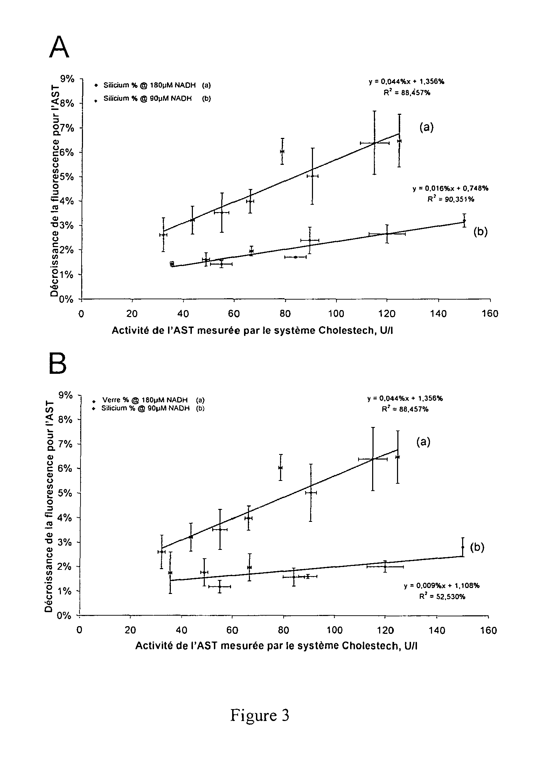

- la Figure 3 représente l'effet de la concentration en NADH sur la mesure de la décroissance de la fluorescence pour le dosage de l'AST. A- Corrélation entre la méthode de référence et la méthode selon la présente invention en utilisant 180µM de NADH (a) ou 90µM de NADH (b). B- Corrélation entre les mesures effectuées avec des chambres microfluidiques dont la base était constituée de verre ou de silicium et avec respectivement 180 µM (a) ou 90 µM (b) de NADH ;

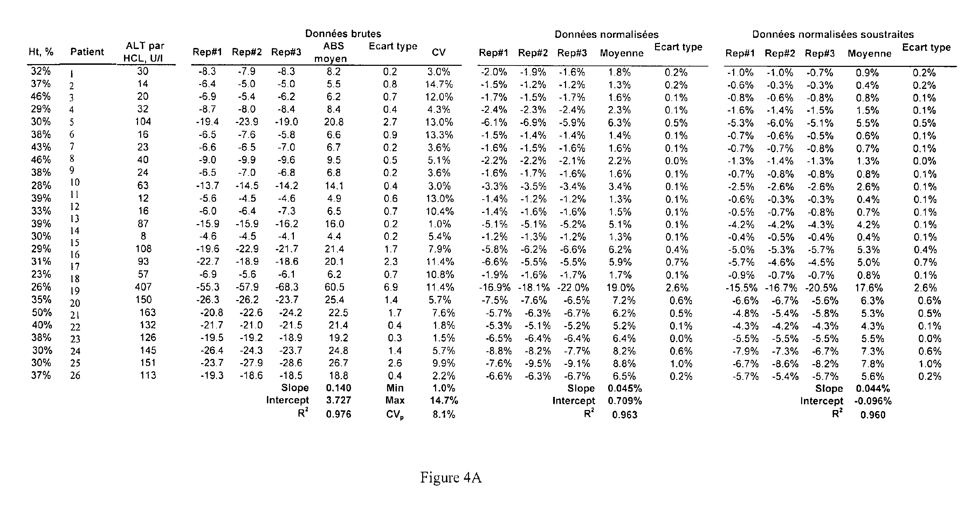

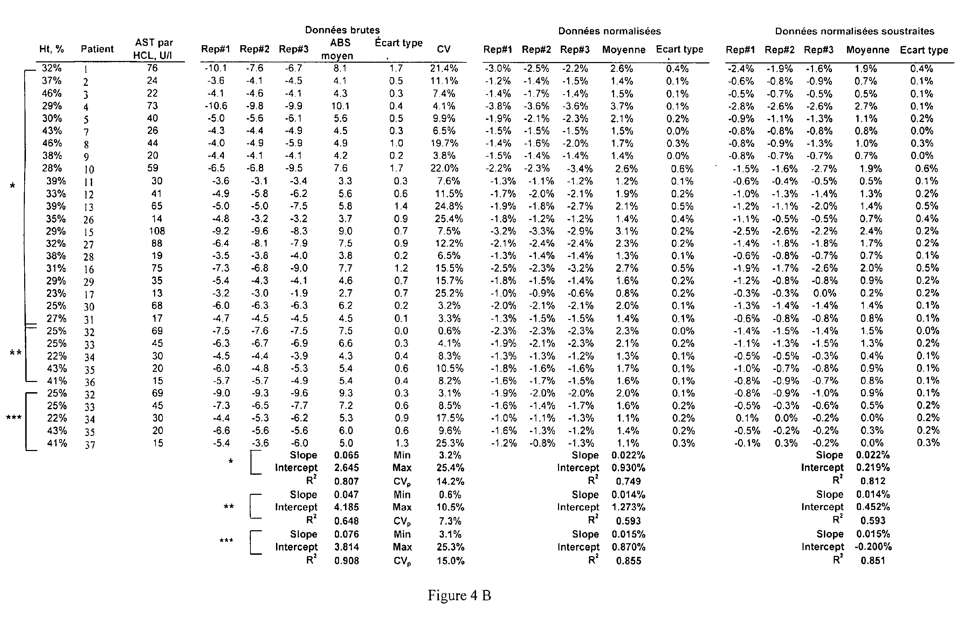

- la Figure 4 représente les résultats détaillés des dosages des transaminases ALT (Figure A) et AST (Figure B) chez des patients hospitalisés. Figures A et B- La valeur « Slope » correspond à la pente de la régression linéaire entre la pente de décroissance de la fluorescence et l'activité transaminase mesurée selon la méthode de référence. La valeur « Intercept » correspond à l'ordonnée à l'origine de cette même régression linéaire. Le « R² » est le coefficient de détermination de la régression linéaire. La valeur « Min » représente la valeur minimale du CV (coefficient de variation), alors que la valeur « Max » représente la valeur maximale du CV. Le « CVₚ »correspond au CV *« poolé* », calculé selon la formule suivante : CVₚ = √((CV₁² + CV₂² + ... + CVₙ²)/n). Ht = hématocrite. ALT ou AST par HCL = activité enzymatique mesurée à l'aide de l'automate de la gamme COBAS (Roche) fonctionnant par mesure de l'absorbance UV du NADH à 339 nm. Rep = répétition. ABS moyen = valeur absolue de la moyenne des 3 expériences. Figure B- Les valeurs marquées * correspondent aux résultats obtenus avec 90 µM de NADH et une phase de latence de 90 secondes. Les valeurs marquées ** correspondent aux résultats obtenus avec 45 µM de NADH et une phase de latence de 90 secondes, et les valeurs marquées *** correspondent aux résultats obtenus avec 90 µM de NADH et une phase de latence de 30 secondes ;

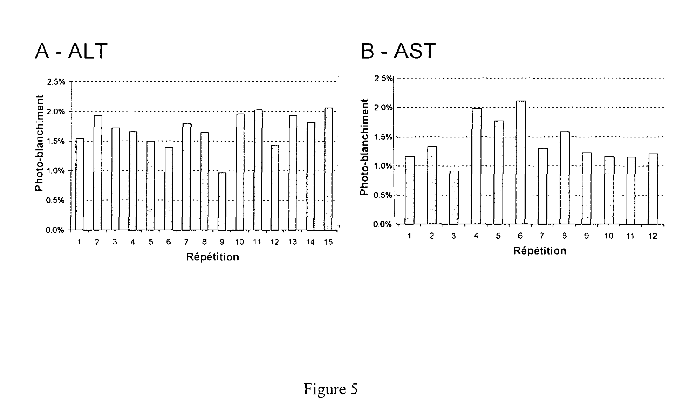

- la Figure 5 représente les valeurs du photo-blanchiment observées pour le dosage des transaminases ALT (figure A) et AST (figure B) par suivi de la décroissance de la fluorescence du NADH ;

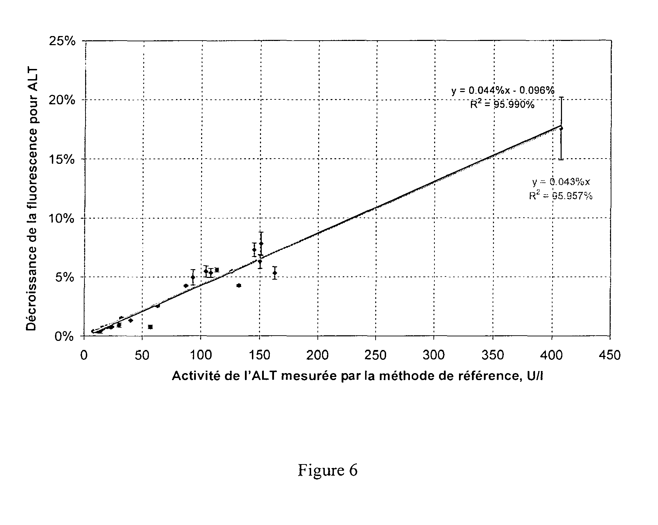

- la Figure 6 est un graphique représentant la corrélation entre la décroissance de la fluorescence du NADH mesurée par la méthode de l'invention et l'activité ALT mesurée par la méthode de référence (automate Cobas). Droite « noire » d'équation y=ax+b ; droite « grise » d'équation y=ax ;

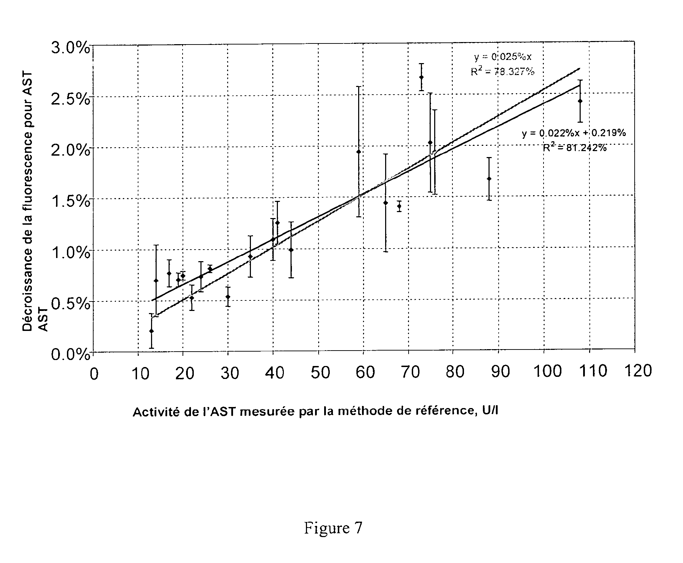

- la Figure 7 est un graphique représentant la corrélation entre la décroissance de la fluorescence du NADH mesurée par la méthode de l'invention et l'activité AST mesurée par la méthode de référence (automate Cobas). Droite « noire » d'équation y=ax+b ; droite « grise » d'équation y=ax ;

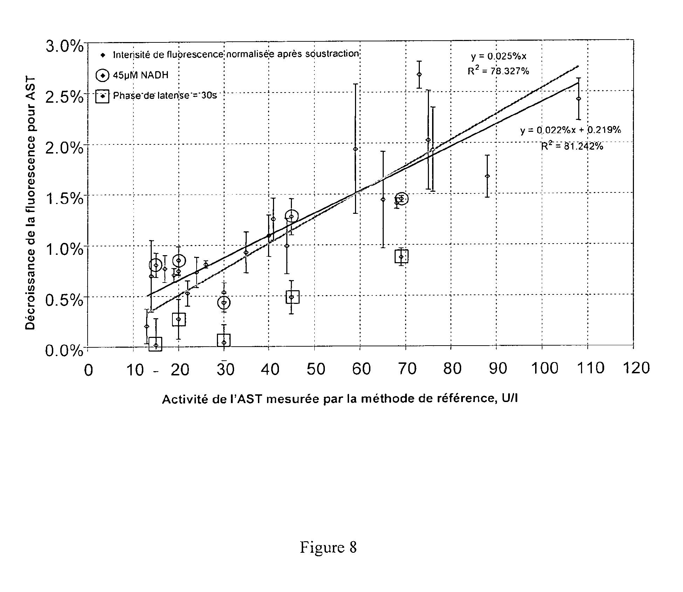

- La Figure 8 est un graphique représentant la corrélation entre la décroissance de la fluorescence du NADH mesurée par la méthode de l'invention et l'activité AST mesurée par la méthode de référence (automate Cobas). La corrélation prend en compte uniquement les valeurs obtenues dans les conditions optimales (90 µM de NADH et une phase de latence de 90 secondes). Les valeurs obtenues avec 45 µM de NADH et les valeurs obtenues avec la phase de latence de 30 s sont cependant reportées sur le graphique afin d'estimer leur adéquation à la droite obtenue. Droite « noire » d'équation y=ax+b ; droite « grise » d'équation y=ax ;

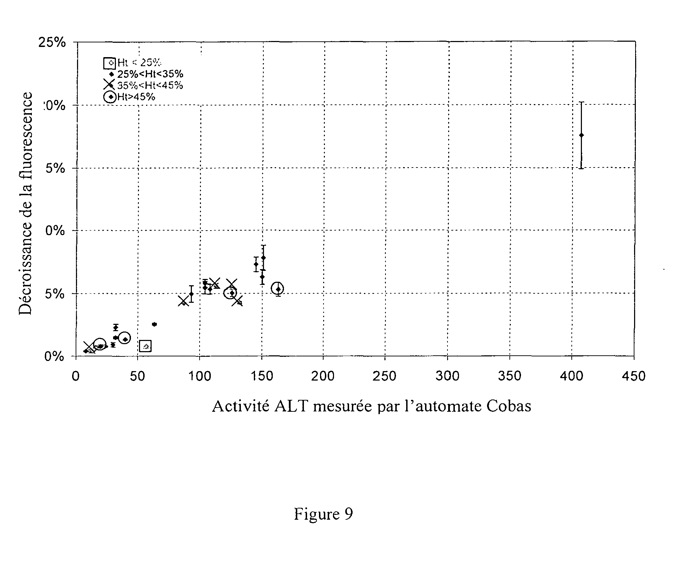

- La Figure 9 est un graphique représentant la corrélation entre la mesure de la décroissance de fluorescence mesurée par la méthode de l'invention et l'activité AST mesurée par la méthode de référence (automate Cobas, Roche) en fonction des différentes classes d'hématocrite (hématocrite < 25% ; 25% < hématocrite < 35% ; 35% < hématocrite < 45% et hématocrite > 45%).

### EXEMPLE 1: DOSAGE DES TRANSAMINASES PAR LA MESURE DE LA DECROISSANCE DE LA FLUORESCENCE DU NADH DANS UN ECHANTILLON DE SANG TOTAL HUMAIN : ETUDE DE DIFFERENTS PARAMETRES POUVANT INFLUER SUR LA FIABILITE ET LA REPETABILITE DES MESURES

### 1.1 Matériels et Méthodes

### a) Réactifs utilisés :

- L-alanine (L-Ala) : exempte d'acide, BioUltra, ≥ 99,5% (NT), Fluka, Réf:05129;
- L-aspartate (L-Asp) : exempt d'acide, BioUltra, ≥ 99,5% (T), Fluka, Réf : 11189 ;
- pyridoxal 5' phosphate monohydrate (PLP) : BioChemika, ≥ 97,0% (NT), Fluka, Réf : 82870 ;
- α-cétoglutarate : sel disodique dihydraté de l'acide α-cétoglutarique, pureté ≥ 98,5% (matière sèche, NT), Fluka, Réf : 75892 ;
- lactate déshydrogénase (LDH) : EC 1.1.1.27, obtenue à partir de muscle squelettique de porc, dans du glycérol, homotétramère, Roche, Réf : 10 127 221 001 ;
- malate déshydrogénase (MDH) : EC 1.1.1.37, obtenue à partir de coeur de porc, dans du glycérol, homodimère, Roche, Réf : 10 127 892 001 ;
- alanine amino transférase (ALT) : Glutamate-Pyruvate Transaminase (GPT), obtenue à partir de coeur de porc, poudre lyophilisée, ≥ 75 unités/mg de protéine, Sigma, Réf : G8255 ;
- aspartate amino transférase (AST) : Glutamate Oxaloacétate Transaminase (GOT), obtenue à partir de coeur de porc, Type II-A, poudre lyophilisée, 100-400 unités/mg de protéine, Sigma, Réf : G7005 ;
- BSA : *« Bovine Serum Albumine* », Fraction V, ≥ 98% (électrophorèse sur gel d'agarose), essentiellement exempte de protéase, poudre lyophilisée, Sigma, Réf: A3294 ;
- Chlorure de Sodium (NaCl) : Sigma, Réf : S9625 ;
- NADH : sel disodique de β-nicotinamide adénine dinucléotide sous forme réduite, Sigma, Réf : N8129 ;
- Tris : Tris(hydroxyméthyl)aminométhane, Sigma, Réf : T1503 ;
- NaN₃ : azide de sodium, ReagentPlus®, ≥ 99%, Sigma-Aldrich, Réf : S2002.

### b) Préparation des solutions pour les réactions enzymatiques : solutions pour le dosage de l'AST (conservées à 4°C) :

- Solution 1 (5 mL) : Tris 96,92 mM pH 7,65, L-Asp 302,4 mM, NaN₃ 8 mM
- Solution 2 (50 µL) : Tris 96,92 mM pH 7,65, NaN₃ 8 mM
- Solution 3 (100 µL) : PLP 6,3 mM
- Solution 4 (50 µL) : NADH 11,34 mM
- Solution 5 (50 µL) : LDH 113,4 U/mL, MDH 75,6 U/mL
- Solution de réaction : Solution 1 + Solution 2 + Solution 3 + Solution 4 + Solution 5

### solutions pour le dosage de l'ALT (conservées à 4°C) :

- Solution 1 (5 mL) : Tris 121,2 mM pH 7,15, L-Ala 630 mM, NaN₃ 8 mM
- Solution 2 (50 µL) ; Tris 121,2 mM pH 7,15, NaN₃ 8 mM
- Solution 3 (100 µL) : PLP 6,3 mM
- Solution 4 (50 µL) : NADH 11,34 mM
- Solution 5 (50 µL) : LDH 214 U/mL
- Solution de réaction : Solution 1 + Solution 2 + Solution 3 + Solution 4 + Solution 5

### c) Fabrication des chambres microfluidiques :

Les chambres fluidiques ont été fabriquées avec une épaisseur de 30 µm (cette épaisseur permet de sédimenter l'ensemble des globules rouges dans la chambre en 90 secondes avant d'effectuer la mesure de la fluorescence). La base des chambres fluidiques était constituée soit d'une lame de verre au format standard de lame de microscope, soit d'une lame de silicium au même format. Du ruban adhésif double face de 30 µm d'épaisseur a ensuite été collé sous forme de plot (pastille) sur la lame de base. Puis une lamelle de microscope en quartz a été collée sur le ruban adhésif, de manière à former la chambre microfluidique. L'utilisation du quartz permet la détection de la fluorescence du NADH tout en étant transparent au rayonnement ultra-violet (λₑₓ du NADH = 320nm-370nm). Les chambres microfluidiques ont été placées sur la platine d'un microscope (Olympus BX60 associé à un filtre optique Oméga XF06), qui se trouvait soit à température ambiante, soit thermostatée à 37°C

### d) Protocole de mesure de la fluorescence

3 µL de sang total humain ont été mélangés à 30 µL de solution de réaction contenant l'ensemble des réactifs nécessaires au dosage (hormis l'α-cétoglutarate). Le mélange ainsi obtenu a été incubé pendant 5 min à 37°C ou à température ambiante. 3µL d'une solution d'α-cétoglutarate (144 mM pour le dosage de l'AST ou 180 mM pour le dosage de l'ALT) ont ensuite été ajoutés au mélange réactionnel afin d'initier la réaction enzymatique. Après une agitation rapide, 20 µL du mélange ont été immédiatement déposés à l'entrée de la chambre microfluidique, qui se remplit par capillarité. Après introduction du mélange réactionnel dans la chambre microfluidique, débute la phase de latence de 90 secondes à 37°C ou à température ambiante. Une fois la phase de latence écoulée, le mélange réactionnel a été excité à une longueur d'onde de 318 (pour le dosage de l'AST) ou 367 nm (pour le dosage de l'ALT), à l'aide d'une lampe UV 100 mW filtrée (bande passante du filtre 320 nm < X < 380 nm), et la cinétique de décroissance du signal de fluorescence a été mesurée durant 180 secondes à l'aide d'une caméra ORCA (Hamamatsu) filtrée (420 nm < λ < 500 nm) ; l'ouverture et la fermeture de l'obturateur et l'acquisition des images se faisant de manière autonome, directement piloté par ordinateur. Durant la cinétique de 180 s, l'acquisition des images se déroulait toutes les 15 s, soit une collecte de 13 photos au total. L'obturateur était ouvert puis fermé à chaque prise d'images, de manière à limiter le photo-blanchiment.

Pour l'expression des résultats, le signal de fluorescence a été normalisé (les données brutes sont normalisées à une même ordonnée (100%) à l'origine) et soustrait (on soustrait à la droite obtenue à partir des données brutes normalisées, la droite obtenue sur un échantillon « blanc » afin de prendre en compte l'effet du photo-blanchiment (*bleaching*))*.*

L'échantillon « blanc » est un échantillon qui ne contient pas de transaminases ou qui contient des transaminases mais dans lequel les réactions catalysées par celles-ci ne sont pas initiées ; il n'y a donc pas de consommation de NADH dans ledit échantillon « blanc ». La seule décroissance de fluorescence qui est alors observée est due au photo-blanchiment.

### e) Méthode de référence

Les résultats obtenus en mettant en oeuvre la méthode de dosage des transaminases selon la présente invention ont été comparés à ceux obtenus avec le système Cholestech LDX® (Cholestech) en suivant le protocole indiqué par le fabricant.

### f) Calcul du photo-blanchiment

Le photo-blanchiment (%) a été calculé sur l'échantillon « blanc » comme suit : [(Intensité de fluorescence de la 1^{ère} mesure - Intensité de fluorescence de la 13^{ème} mesure) / (Intensité de fluorescence de la 1^{ère} mesure)] x 100.

### 1.2 Résultats

### 1.2.1. Effet de la température

L'effet de la température sur les résultats obtenus a été vérifié. Pour cela, un échantillon de sang total provenant d'un donneur sain de l'Etablissement Français du Sang (EFS) a été supplémenté avec des transaminases porcines pour obtenir une gamme de concentration en transaminases, à savoir entre 10 et 100 U/L pour l'AST et entre 20 et 200 U/L pour l'ALT. Le dosage selon le système Cholestech LDX® des 8 échantillons de la gamme préparée montrait des activités enzymatiques comprises entre 34 et 110 U/L pour l'AST, et entre 20 et 177 U/L pour l'ALT. Ces mêmes échantillons ont ensuite été analysés par la méthode selon la présente invention permettant de suivre la décroissance de la fluorescence du NADH, soit à température ambiante (22°C), soit à 37°C, et en utilisant des chambres microfluidiques dont la base était constituée de verre. Les blancs effectués pour cette expérience ont été réalisés avec un culot de globules rouges lavés de manière à éliminer toute trace d'activité catalytique résiduelle des transaminases plasmatiques.

Les résultats pour le dosage de l'ALT sont présentés à la Figure 1. La Figure 1A montre les corrélations obtenues entre la méthode de référence (système Cholestech LDX®) et la décroissance de la fluorescence après normalisation des données et soustraction de l'échantillon « blanc ». Les deux corrélations montrent des coefficients R²=0,978 à 22°C (température ambiante) et R²=0,951 à 37°C. Il ressort de cette Figure que les décroissances de la fluorescence observées à 37°C sont plus importantes que celles observées à température ambiante, ce qui est tout à fait cohérent car les réactions enzymatiques sont optimales à 37°C. La corrélation entre les mesures effectuées à 37°C, et celles effectuées à température ambiante, illustrée à la Figure 1B, montre cette amélioration de résultats : les valeurs sont augmentées d'un facteur 1,66 à 37°C par rapport à la température ambiante.

Pour cette série d'expériences à différentes températures, le photo-blanchiment ne semble pas être affecté par la température. Ceci est cohérent dans la mesure où le photo-blanchiment du fluorophore est dû essentiellement au temps d'exposition du faisceau lumineux et non à la température.

La mesure de la décroissance de la fluorescence du NADH permettant le suivi de l'activité enzymatique des transaminases peut donc être effectuée à 37°C ou à température ambiante, même si la température de 37°C est optimale.

### 1.2.2. Effet du matériau de la base de la chambre microfluidique (verre ou silicium)

Les diminutions de signal de fluorescence observées dans des chambres microfluidiques en verre se sont avérées être de 10% au maximum pour les taux de transaminases les plus élevés (soit 200 U/L pour l'ALT). Aux longueurs d'onde d'excitation utilisées (comprises entre 300 et 400 nm), le verre est absorbant. Une partie du signal d'excitation peut donc être perdue par absorption par le verre. L'utilisation d'un matériau réfléchissant permettrait de pallier à cette perte. Il a donc été étudié s'il était possible d'augmenter ces décroissances de signal au delà de cette limite de 10%, de manière à améliorer la précision des mesures.

Des chambres microfluidiques comprenant une base en silicium ont donc été fabriquées tel que décrit au paragraphe 1.1.c) ci-dessus. Un échantillon de sang provenant d'un donneur sain de l'EFS a été supplémenté avec des transaminases porcines (voir paragraphe 1.2.1). Le dosage selon le système Cholestech LDX® des 8 échantillons de la gamme préparée montraient des activités enzymatiques comprises entre 35 et 108 U/L pour l'AST, et entre 27 et 223 U/L pour l'ALT. Ces mêmes échantillons ont ensuite été dosés selon la méthode de la présente invention à 37°C, dans des chambres microfluidiques en verre ou en silicium. Les blancs effectués pour cette expérience ont été réalisés avec un culot de globules rouges lavés.

Les résultats pour le dosage de l'ALT sont présentés à la Figure 2. La Figure 2A montre les corrélations obtenues entre la méthode de référence et la mesure de la décroissance de la fluorescence (selon la méthode de la présente invention) après normalisation des données et soustraction du blanc. Les CV observés pour les expériences réalisées avec les bases des chambres microfluidiques en verre sont compris entre 1,8% et 29,6%, alors que ceux obtenus avec les bases des chambres microfluidiques en silicium sont compris entre 1,8 et 14,5% : la répétabilité est donc améliorée avec le silicium. Par ailleurs, il ressort de cette figure que les décroissances de la fluorescence observées avec les bases des chambres microfluidiques en silicium sont plus importantes que celles observées sur verre. La corrélation entre les mesures effectuées avec les bases des chambres microfluidiques en verre, et celles effectuées avec les bases des chambres microfluidiques en silicium, illustrée à la Figure 2B, montre cette amélioration de résultats : les valeurs sont augmentées d'un facteur 1,6 avec le silicium par rapport au verre.

Le photo-blanchiment est inférieur à 4% avec le verre, alors qu'il est inférieur à 7% avec silicium. Ceci s'explique par le fait que le faisceau traverse deux fois l'épaisseur de la chambre microfluidique, puisqu'il est réfléchi : le photo-blanchiment s'en trouve ainsi augmenté.

Le verre et le silicium peuvent donc être utilisés pour la fabrication de la base des chambres microfluidiques. Cependant, l'utilisation du silicium permet d'obtenir des diminutions de signal plus importantes par rapport à l'utilisation du verre, et la répétabilité des mesures en est améliorée.

### 1.2.3. Effet de la concentration en NADH

Un échantillon de sang provenant d'un donneur sain de l'EFS a été supplémenté avec des transaminases porcines à 8 taux différents de manière à établir une gamme d'activité enzymatique. Le dosage des transaminases a été effectué avec des concentrations de NADH de 90 µM ou 180 µM. Le dosage selon le système Cholestech LDX® des 8 échantillons de la gamme préparée montrait des activités enzymatiques comprises entre 32 et 125 U/L pour l'AST, et entre 19 et 228 U/L pour l'ALT. Ces mêmes échantillons ont ensuite été analysés par la méthode selon la présente invention à 37°C, avec des chambres microfluidiques dont la base était constituée de silicium. Les blancs effectués pour cette expérience ont été réalisés avec un culot de globules rouges lavés.

Les résultats du dosage de l'AST sont présentés à la Figure 3. La Figure 3A montre les corrélations obtenues entre la méthode de référence et celle selon la présente invention après normalisation des données. Les CV observés aux deux concentrations de NADH sont comparables, systématiquement inférieurs à 30%. Les deux corrélations montrent des coefficients R² similaires, R²=0,903 avec 180µM de NADH et R²=0,885 avec 90 µM de NADH. Il ressort de cette figure que la décroissance de la fluorescence observée avec 90 µM de NADH est plus importante que celle observée avec 180µM de NADH. Les pentes des régressions linéaires varient d'un facteur 2,75.

La Figure 3B illustre l'amélioration entre la configuration initiale, chambre en verre et 180 µM de NADH, et la configuration optimale, chambre en silicium et 90 µM de NADH. Les variations de décroissance de la fluorescence ont été améliorées quasiment d'un facteur 5 entre les deux configurations.

Pour le dosage de l'AST, l'utilisation de 90 µM de NADH s'avère plus fiable, car permettant d'obtenir des variations de signal de décroissance de la fluorescence plus importantes.

### EXEMPLE 2: VALIDATION EXPERIMENTALE DE LA METHODE DE DOSAGE DES TRANSAMINASES PAR LA MESURE DE LA FLUORESCENCE DU NADH

Les résultats présentés à l'Exemple 1 concernaient des dosages effectués sur des échantillons de sang provenant de donneurs sains, qui ont été supplémentés en transaminases porcines afin de mimer des échantillons de patients malades, dont les taux de transaminases seraient élevés. La méthode de dosage des transaminases selon la présente invention a ensuite été validée sur des échantillons de sang total de patients malades prélevés sur citrate.

Des échantillons de sang citraté provenant de patients hospitalisés aux Hospices Civils de Lyon (HCL, France) ont été analysés selon la méthode de la présente invention. Les mesures de la fluorescence du NADH ont été effectuées selon la méthode décrite à l'Exemple I, avec les paramètres suivants :
- une température de 37°C ;
- des chambres microfluidiques avec une base en silicium ;
- une concentration de NADH de 180 µM pour le dosage de l'ALT et de 90 µM pour le dosage de l'AST ;
- des mesures réalisées en triplicatat ;
- les « blancs » ont été réalisés en remplaçant l'α-cétoglutarate (qui initie la réaction enzymatique) par un volume équivalent de NaCl 154 mM.

Les résultats obtenus par la mesure de décroissance de la fluorescence selon la méthode de la présente invention ont été comparés à ceux obtenus par la méthode de référence mise en oeuvre au sein du laboratoire de Biochimie de l'hôpital Edouard Herriot de Lyon (France). Le Tableau 1 ci-dessous décrit les principales caractéristiques des deux méthodes :

**Tableau 1 : comparaison de la méthode de dosage des transaminases AST/ALT mise en oeuvre par le laboratoire de Biochimie de l'hôpital Edouard Herriot de Lyon à l'aide d'un automate de la gamme Cobas (Roche) et la méthode selon la présente invention.**

| | Méthode de référence | Méthode de l'invention |
|---|---|---|
| Système de mesure | Automate de la gamme Cobas de la société Roche fonctionnant par mesure de l'absorbance UV à 339 nm (décroissance du NADH en présence de PLP) | Banc expérimental permettant la mesure de la décroissance de la fluorescence du NADH à 460nm (voir Exemple 1.1) |
| Volume de l'échantillon | 10µL de plasma hépariné prélevé dans un tube de 5mL de sang centrifugé (3µL de plasma si taux fort, 40µL de plasma si taux faible) | 3µL de sang total citraté |
| Température | 37°C | 37°C |
| Nombre réplicats | 1 | 3 |
| Gamme | 4-600U/L pour ALT | 20-200U/L pour ALT |
| | 4-800U/L pour AST | 10-100U/L pour AST |
| Durée de l'analyse | ~ 20 min de centrifugation + 10 min de passage dans l'automate | 9 min 30 pour une seule mesure |
| | ~ 30 min pour une seule mesure | |
| Reproductibilité | CV < 4% (intra et inter-série) pour ALT et AST | CV < 15% pour ALT et CV < 25% pour AST |

25 échantillons de sang provenant de patients différents ont été analysés pour le dosage de l'ALT, et 21 échantillons de sang provenant de patients différents ont été analysés pour le dosage de l'AST. Les résultats bruts sont représentés à la Figure 4.

### 2.1. Estimation du photo-blanchiment

Durant la cinétique des réactions enzymatiques (180 secondes), des photos ont été prises toutes les 15 secondes, avec ouverture puis fermeture de l'obturateur à chaque prise. Un objectif 10X a été utilisé et les temps d'exposition étaient de 50 millisecondes pour un facteur d'amplification électronique du signal optique de 250 (qui est détecté au niveau de la caméra).

Le photo-blanchiment (calculé selon la méthode décrite à l'Exemple 1.1.f) ci-dessus) a été estimé sur les mesures réalisées sur les échantillons « blancs ». Les résultats, présentés à la Figure 5 montrent que le photo-blanchiment est systématiquement inférieur à 2% pour cette série d'expérience où les échantillons « blancs » sont réalisés en remplaçant l'α-cétoglutarate par du NaCl 154 mM.

### 2.2. Corrélation entre la méthode de dosage de référence et la méthode de dosage selon la présente invention

### 2.2.1. pour l'ALT :

Les résultats obtenus sont présentés à la Figure 6. La corrélation est linéaire. Elle est correcte sur l'ensemble de la gamme étudiée, avec des coefficients de régression linéaire supérieurs à 0,959.

### 2.2.2 pour l'AST:

Les résultats obtenus sont présentés à la Figure 7. La corrélation est linéaire avec des R² de 0,812 et 0,783 pour respectivement une régression linéaire de type y=ax+b et une régression linéaire de type y=ax. On note également que les variations de signal observées pour le dosage de l'AST sont 2 fois plus faibles que celles déterminées pour l'ALT. Par exemple, pour une activité transaminase de 100 U/L, la décroissance de la fluorescence pour ALT est de 5%, alors qu'elle n'est que de 2,5% pour l'AST.

Il a donc été vérifié s'il était possible d'améliorer cette décroissance de signal en testant deux paramètres susceptibles d'influencer ces résultats : la quantité de NADH (45 µM au lieu de 90 µM) et la phase de latence (30 s au lieu de 90 s). Les résultats obtenus sont présentés à la Figure 8.

Il ressort du graphique de la Figure 8 que la diminution de la concentration de NADH de 90 µM à 45 µM ne permet pas d'augmenter la décroissance de la fluorescence : les valeurs obtenues sont du même ordre de grandeur que celles observées avec 90 µM de NADH. Il est cependant intéressant de noter que les CV sont meilleurs avec 45 µM de NADH : en effet, dans ces conditions, les valeurs des 5 CV varient entre 0,6% et 10,5%. Ainsi, même si la diminution de la concentration ne permet pas d'obtenir des décroissances de la fluorescence plus importantes, elle permet une meilleure répétabilité.

Par ailleurs, une phase de latence de 30 s au lieu de 90 s, n'a pas permis d'améliorer les résultats. Au contraire, les décroissances de la fluorescence étaient plus faibles dans ces conditions.

### 2.3. Effet de l'hématocrite

L'hématocrite pourrait constituer un paramètre impactant les mesures de la fluorescence. En effet, la mesure de la fluorescence du NADH est effectuée en sang total, donc en présence des globules rouges. Des taux variables d'hématocrites chez divers patients présentant par ailleurs un taux de transaminases identique pourrait donc donner des résultats de dosage de transaminases différents à cause de la variation de l'absorption due aux quantités variables de globules rouges.

Afin de vérifier cette hypothèse, il a été testé l'effet de l'hématocrite sur les mesures de la décroissance de fluorescence. Les différents patients qui ont été analysés présentaient des taux d'hématocrite compris entre 21,6 % et 51 % (moyenne = 34,3%, écart-type = 7,3%). Compte-tenu de la gamme d'hématocrite disponible, différentes classes ont été définies :
- hématocrite < 25%
- 25% < hématocrite < 35%
- 35% < hématocrite < 45%
- hématocrite > 45%

Les résultats obtenus sont présentés à la Figure 9. Ces résultats ne mettent pas en évidence de tendance entre les différentes valeurs de décroissance de fluorescence mesurées et la valeur de l'hématocrite. On peut seulement noter que le patient présentant le taux d'hématocrite le plus faible semble être en dehors de la corrélation.

On n'observe donc pas d'effet de l'hématocrite sur les mesures de décroissance de la fluorescence du NADH.

## Revendications

1. Méthode de dosage, dans un échantillon de liquide biologique, d'une enzyme catalytique plasmatique NADH-dépendante (E_{N}), par mesure de son activité enzymatique, comprenant une étape de mesure de la décroissance de la fluorescence du NADH, laquelle méthode est **caractérisée en ce que** ledit liquide biologique est du sang total et **en ce que** l'activité enzymatique de ladite enzyme catalytique est mesurée en suivant la décroissance de la fluorescence du NADH dans une chambre microfluidique de 20 µm à 2 mm d'épaisseur.

2. Méthode selon la revendication 1, **caractérisée en ce que** ladite enzyme catalytique est choisie dans le groupe constitué par la lactate déshydrogénase (LDH), la malate déshydrogénase (MDH) et l'isocitrate déshydrogénase (IDH).

3. Méthode de dosage, dans un échantillon de liquide biologique, d'une enzyme catalytique plasmatique (E_{C}) ayant comme substrats S₁ et S₂, par mesure de l'activité enzymatique de ladite enzyme E_{C}, ladite enzyme E_{C} étant une enzyme catalytique plasmatique dont l'un des produits obtenus au cours de la réaction enzymatique catalysée par celle-ci est lui-même un substrat d'au moins une enzyme catalytique NADH-dépendante (E_{N}) ayant comme cofacteur le NADH, ladite méthode comprenant au moins les étapes suivantes : a) incubation dudit liquide biologique avec ledit substrat S₁, du NADH, une ou plusieurs enzyme(s) catalytique(s) NADH-dépendante(s) (E_{N}) différentes ayant chacune pour substrat l'un des produits de la réaction enzymatique catalysée par E_{C}, et optionnellement du phosphate de pyridoxal (PLP) ; b) ajout dudit substrat S₂ de manière à initier les réactions enzymatiques catalysées par les enzymes catalytiques E_{C} et EN, laquelle méthode est **caractérisée :**
- **en ce que** ledit liquide biologique est du sang total,
- **en ce qu'**après l'ajout du substrat S₂ à l'étape b), le mélange obtenu est immédiatement introduit dans une chambre microfluidique de 20 µm à 2 mm d'épaisseur, et
- **en ce que** l'activité enzymatique de ladite enzyme catalytique E_{C} est mesurée en suivant la décroissance de la fluorescence du NADH dans ladite chambre microfluidique.

4. Méthode selon l'une quelconque des revendications 1 à 3, **caractérisée en ce que** l'épaisseur de ladite chambre microfluidique est comprise entre 30 µm et 50 µm.

5. Méthode selon la revendication 4, **caractérisée en ce que** l'épaisseur de ladite chambre microfluidique est de 30 µm.

6. Méthode selon l'une quelconque des revendications 1 à 5, **caractérisée en ce que** ladite chambre microfluidique comprend deux parois sensiblement parallèles et séparées par un ou plusieurs moyens d'espacement tel(s) que la distance entre lesdites deux parois soit comprise entre 20 µm à 2 mm.

7. Méthode selon la revendication 6, **caractérisée en ce que** la distance entre lesdites deux parois est comprise entre 30 µm à 50 µm.

8. Méthode selon la revendication 6 ou 7, **caractérisée en ce qu'**au moins une des deux parois est constituée d'un matériau réfléchissant la lumière de fluorescence.

9. Méthode selon la revendication 8, caractérisée en ce ledit matériau réfléchissant est du silicium ou un oxyde ou un nitrure de silicium.

10. Méthode selon l'une quelconque des revendications 1 à 9, **caractérisée en ce que** la mesure de la décroissance de la fluorescence s'effectue après l'écoulement d'un temps de latence défini par le temps de sédimentation des globules rouges dans ladite chambre microfluidique.

11. Méthode selon l'une quelconque des revendications 1 à 10, **caractérisée en ce que** la fluorescence du NADH est mesurée à une longueur d'onde comprise entre 420 nm et 550 nm, après excitation du NADH à une longueur comprise entre 300 et 400 nm.

12. Méthode selon l'une quelconque des revendications 1 à 11, **caractérisée en ce que** le volume de l'échantillon de sang total est compris entre 0,1 µL et 50 µL.

13. Méthode selon l'une quelconque des revendications 3 à 12, **caractérisée en ce que** le temps d'incubation à l'étape a) est compris entre 2 et 30 minutes.

14. Méthode selon la revendication 13, **caractérisée en ce que** le temps d'incubation à l'étape a) est de 5 minutes.

15. Méthode selon l'une quelconque des revendications 3 à 14, **caractérisée en ce que** la concentration du NADH dans le mélange obtenu à l'étape a) est de 20 µM à 250 µM.

16. Méthode selon l'une quelconque des revendications 3 à 15, **caractérisée en ce que** ladite enzyme catalytique E_{C} est choisie dans le groupe constitué par l'aspartate amino transférase (AST), l'alanine amino transférase (ALT), la pyruvate kinase, la créatine kinase et la glycérol kinase.

17. Méthode selon la revendication 16, **caractérisée en ce que** ladite enzyme catalytique E_{C} est choisie dans le groupe constitué par l'AST et l'ALT.

18. Méthode selon l'une quelconque des revendications 3 à 17, **caractérisée en ce que** ladite enzyme catalytique E_{C} est l'aspartate amino transférase, ledit substrat S₁ est le L-aspartate, ledit substrat S₂ est l'α-cétoglutarate, et lesdites enzymes catalytiques EN sont choisies parmi soit une malate déshydrogénase (MDH), soit une MDH et une lactate déshydrogénase (LDH).

19. Méthode selon l'une quelconque des revendications 3 à 17, **caractérisée en ce que** ladite enzyme catalytique E_{C} est l'alanine amino transférase, ledit substrat S₁ est la L-alanine, ledit substrat S₂ est l'α-cétoglutarate, et ladite enzyme catalytique EN est une lactate déshydrogénase.

20. Utilisation d'une chambre microfluidique telle que définie à l'une quelconque des revendications 1 et 4 à 9, pour doser une enzyme catalytique plasmatique dans un échantillon de sang total.

21. Utilisation selon la revendication 20, **caractérisée en ce que** ladite enzyme catalytique est choisie dans le groupe constitué par l'AST, l'ALT, la pyruvate kinase, la créatine kinase, la glycérol kinase, la LDH, la MDH et l'IDH.

22. Kit ou coffret pour doser une enzyme catalytique plasmatique (E_{C}) dans un échantillon de sang total, ladite enzyme E_{C} étant une enzyme catalytique plasmatique dont l'un des produits obtenus au cours de la réaction enzymatique catalysée par celle-ci est lui-même un substrat d'au moins une enzyme catalytique NADH-dépendante (E_{N}) ayant comme cofacteur le NADH, et ladite enzyme E_{C} ayant comme substrats S₁ et S₂, ledit substrat S₁ étant le L-aspartate et ledit substrat S₂ étant l'α-cétoglutarate, ou ledit substrat S₁ étant la L-alanine et ledit substrat S₂ étant l'α-cétoglutarate, **caractérisé en ce qu'**il comprend une chambre microfluidique telle que définie à l'une quelconque des revendications 1 et 4 à 9, et les réactifs suivants : les substrats S₁ et S₂, le NADH, au moins une enzyme catalytique NADH-dépendante (E_{N}), et optionnellement du PLP.

## Patentansprüche

1. Verfahren zur Bestimmung, in einer Probe von biologischer Flüssigkeit, eines katalytischen plasmatischen NADH-abhängigen Enzyms (E_{N}) durch Messung seiner katalytischen Aktivität, das eine Stufe der Messung der Abnahme der Fluoreszenz des NADH umfasst, wobei das Verfahren **dadurch gekennzeichnet ist, dass** die biologische Flüssigkeit Vollblut ist und dadurch, dass die enzymatische Aktivität des katalytischen Enzyms gemessen wird, indem die Abnahme der Fluoreszenz des NADH in einer Mikrofluidkammer mit einer Dicke von 20 µm bis 2 mm verfolgt wird.

2. Verfahren gemäß Anspruch 1, **dadurch gekennzeichnet, dass** das katalytische Enzym aus der Gruppe, bestehend aus Lactatdehydrogenase (LDH), Malatdehydrogenase (MDH) und Isocitratdehydrogenase (IDH), ausgewählt wird.

3. Verfahren zur Bestimmung, in einer Probe von biologischer Flüssigkeit, eines katalytischen plasmatischen Enzyms (E_{C}), das als Substrate S₁ und S₂ hat, durch Messung der enzymatischen Aktivität des Enzyms E_{C}, wobei das Enzym E_{C} ein katalytisches plasmatisches Enzym ist, wobei eins der Produkte, die im Verlauf der katalysierten enzymatischen Reaktion durch dieses erhalten werden, selbst ein Substrat wenigstens eines katalytischen NADH-abhängigen Enzyms (E_{N}) ist, das als Cofaktor NADH hat, wobei das Verfahren wenigstens die folgenden Stufen umfasst: a) Inkubation der biologischen Flüssigkeit mit dem Substrat S₁, NADH, einem katalytischen NADH-abhängigen Enzym oder mehreren katalytischen NADH-abhängigen Enzymen (E_{N}), die unterschiedlich sind, von denen jedes eins der Produkte der durch E_{C} katalysierten enzymatischen Reaktion als Substrat hat, und gegebenenfalls Pyridoxalphosphat (PLP); b) Zusatz des Substrates S₂ derart, dass die enzymatischen Reaktionen, die durch die katalytischen Enzyme E_{C} und E_{N} katalysiert werden, initiiert werden, wobei das Verfahren **dadurch gekennzeichnet ist:**
- **dass** die biologische Flüssigkeit Vollblut ist,
- **dass** nach Zusatz des Substrates S₂ in der Stufe b) das erhaltene Gemisch unmittelbar in eine Mikrofluidkammer mit einer Dicke von 20 µm bis 2 mm eingeführt wird und
- **dass** die enzymatische Aktivität des katalytischen Enzyms E_{C} gemessen wird, indem die Abnahme der Fluoreszenz des NADH in der Mikrofluidkammer verfolgt wird.

4. Verfahren gemäß einem der Ansprüche 1 bis 3, **dadurch gekennzeichnet, dass** die Dicke der Mikrofluidkammer zwischen 30 µm und 50 µm liegt.

5. Verfahren gemäß Anspruch 4, **dadurch gekennzeichnet, dass** die Dicke der Mikrofluidkammer 30 µm ist.

6. Verfahren gemäß einem der Ansprüche 1 bis 5, **dadurch gekennzeichnet, dass** die Mikrofluidkammer zwei Wände umfasst, die etwa parallel sind und durch ein oder mehrere Abstandsmittel getrennt sind, so dass der Abstand zwischen den zwei Wänden zwischen 20 µm und 2 mm liegt.

7. Verfahren gemäß Anspruch 6, **dadurch gekennzeichnet, dass** der Abstand zwischen den zwei Wänden zwischen 30 µm und 50 µm liegt.

8. Verfahren gemäß Anspruch 6 oder 7, **dadurch gekennzeichnet, dass** wenigstens eine der zwei Wände aus einem Material besteht, das das Fluoreszenzlicht reflektiert.

9. Verfahren gemäß Anspruch 8, **dadurch gekennzeichnet, dass** das Material, das reflektiert, Silicium oder ein Oxid oder ein Nitrid von Silicium ist.

10. Verfahren gemäß einem der Ansprüche 1 bis 9, **dadurch gekennzeichnet, dass** die Messung der Abnahme der Fluoreszenz nach Ablauf einer Latenzzeit erfolgt, welche durch die Sedimentationszeit der Erythrozyten in der Mikrofluidkammer definiert ist.

11. Verfahren gemäß einem der Ansprüche 1 bis 10, **dadurch gekennzeichnet, dass** die Fluoreszenz des NADH bei einer Wellenlänge von zwischen 420 nm und 550 nm nach Anregung des NADH bei einer Wellenlänge zwischen 300 und 400 nm gemessen wird.

12. Verfahren gemäß einem der Ansprüche 1 bis 11, **dadurch gekennzeichnet, dass** das Volumen der Vollblutprobe zwischen 0,1 µl und 50 µl liegt.

13. Verfahren gemäß einem der Ansprüche 3 bis 12, **dadurch gekennzeichnet, dass** die Inkubationszeit in der Stufe a) zwischen 2 und 30 Minuten liegt.

14. Verfahren gemäß Anspruch 13, **dadurch gekennzeichnet, dass** die Inkubationszeit in der Stufe a) 5 Minuten ist.

15. Verfahren gemäß einem der Ansprüche 3 bis 14, **dadurch gekennzeichnet, dass** die Konzentration des NADH in dem in der Stufe a) erhaltenen Gemischs von 20 µM bis 250 µM ist.

16. Verfahren gemäß einem der Ansprüche 3 bis 15, **dadurch gekennzeichnet, dass** das katalytische Enzym E_{C} aus der Gruppe, bestehend aus Aspartataminotransferase (AST), Alaninaminotransferase (ALT), Pyruvatkinase, Kreatinkinase und Glycerinkinase, ausgewählt wird.

17. Verfahren gemäß Anspruch 16, **dadurch gekennzeichnet, dass** das katalytische Enzym E_{C} aus der Gruppe, bestehend aus AST und ALT, ausgewählt wird.

18. Verfahren gemäß einem der Ansprüche 3 bis 17, **dadurch gekennzeichnet, dass** das katalytische Enzym E_{C} Aspartataminotransferase ist, das Substrat S₁ L-Aspartat ist, das Substrat S₂ α-Ketoglutarat ist, und die katalytischen Enzyme E_{N} aus Malatdehydrogenase (MDH) oder MDH und Lactatdehydrogenase (LDH) ausgewählt werden.

19. Verfahren gemäß einem der Ansprüche 3 bis 17, **dadurch gekennzeichnet, dass** das katalytische Enzym E_{C} Alaninaminotransferase ist, das Substrat S₁ L-Alanin ist, das Substrat S₂ α-Ketoglutarat ist und das katalytische Enzym E_{N} Lactatdehydrogenase ist.

20. Verwendung einer Mikrofluidkammer, wie sie in einem der Ansprüche 1 und 4 bis 9 definiert ist, um ein katalytisches plasmatisches Enzym in einer Vollblutprobe zu bestimmen.

21. Verwendung gemäß Anspruch 20, **dadurch gekennzeichnet, dass** das katalytische Enzym aus der Gruppe, bestehend aus AST, ALT, Pyruvatkinase, Kreatinkinase, Glycerinkinase, LDH, MDH und IDH ausgewählt ist.

22. Kit oder Behältnis zum Dosieren eines katalytischen plasmatischen Enzyms (E_{C}) in einer Vollblutprobe, wobei das Enzym E_{C} ein katalytisches plasmatisches Enzym ist, wobei eins der Produkte, die im Verlauf der enzymatischen durch dieses katalysierten Reaktion erhalten werden, selbst ein Substrat wenigstens eines katalytischen NADH-abhängigen Enzyms (E_{N}) ist, welches als Cofaktor NADH hat, und wobei das Enzym E_{C} als Substrate S₁ und S₂ hat, wobei das Substrat S₁ L-Aspartat ist und das Substrat S₂ α-Ketoglutarat ist oder das Substrat S₁ L-Alanin ist und das Substrat S₂ α-Ketoglutarat ist, **dadurch gekennzeichnet, dass** er/es eine Mikrofluidkammer wie sie in einem der Ansprüche 1 und 4 bis 9 definiert ist, und die folgenden Reagenzien umfasst: die Substrate S₁ und S₂, NADH, wenigstens ein katalytisches NADH-abhängiges Enzym (E_{N}) und gegebenenfalls PLP.

## Claims

1. A method for assaying, in a biological liquid sample, an NADH-dependent catalytic plasma enzyme (E_{N}), by measuring its enzymatic activity, comprising a step of measuring the decrease in the fluorescence of the NADH, said method is **characterized in that** said biological liquid is whole blood and the enzymatic activity of said catalytic enzyme is measured by monitoring the decrease in the fluorescence of the NADH in a microfluidic chamber having a thickness of 20 µm to 2 mm.

2. The method as claimed in claim 1, **characterized in that** said catalytic enzyme is chosen from the group consisting of lactate dehydrogenase (LDH), malate dehydrogenase (MDH) and isocitrate dehydrogenase (IDH).

3. A method for assaying, in a sample of biological liquid, a catalytic plasma enzyme (E_{C}) having S₁ and S₂ as substrates, by measuring the enzymatic activity of said enzyme E_{C}, said enzyme (E_{C}) being a catalytic plasma enzyme for which one of the products obtained during the enzymatic reaction catalyzed by it is itself a substrate of at least one NADH-dependent catalytic enzyme (E_{N}) having NADH as cofactor, said method comprising at least the following steps: a) incubation of said biological liquid with said substrate S₁, NADH, one or more different NADH-dependent catalytic enzymes (E_{N}) each having as substrate one of the products of the enzymatic reaction catalyzed by E_{C}, and optionally pyridoxal phosphate (PLP); b) addition of said substrate S₂ so as to initiate the enzymatic reactions catalyzed by the catalytic enzymes E_{C} and E_{N}; said method in **characterized:**
- **in that** said biological liquid is whole blood,
- **in that** after the addition of the substrate S₂ in step b), the mixture obtained is immediately introduced into a microfluidic chamber 20 µm to 2 mm thick, and
- **in that** the enzymatic activity of said catalytic enzyme E_{C} is measured by monitoring the decrease in the fluorescence of NADH in said microfluidic chamber.

4. The method as claimed in any one of claims 1 to 3, **characterized in that** the thickness of said microfluidic chamber is between 30 µm and 50 µm.

5. The method as claimed in claim 4, **characterized in that** the thickness of said microfluidic chamber is 30 µm.

6. The method as claimed in any one of claims 1 to 5, **characterized in that** said microfluidic chamber comprises two walls, substantially parallel and separated by one or more means of keeping apart, such that the distance between said two walls is between 2 µm and 2 mm.

7. The method as claimed in claim 6, **characterized in that** the distance between said two walls is between 30 µm and 50 µm.

8. The method as claimed in claim 6 or claim 7, **characterized in that** at least one of the two walls consists of a material reflecting fluorescence light.

9. The method as claimed in claim 8, **characterized in that** said reflective material is silicon or a silicon oxide or nitride.

10. The method as claimed in any one of claims 1 to 9, **characterized in that** the measurement of the decrease in fluorescence is carried out after a latent time has elapsed, defined by the sedimentation time of the red blood cells in said microfluidic chamber.

11. The method as claimed in any one of claims 1 to 10, **characterized in that** the fluorescence of NADH is measured at a wavelength of between 420 nm and 550 nm, after excitation of the NADH at a wavelength of between 300 and 400 nm.

12. The method as claimed in any one of claims 1 to 11, **characterized in that** the volume of the whole-blood sample is between 0.1 µL and 50 µL.

13. The method as claimed in any one of claims 3 to 12, **characterized in that** the incubation time in step a) is between 2 and 30 minutes.

14. The method as claimed in claim 13, **characterized in that** the incubation time in step a) is 5 minutes.

15. The method as claimed in any one of claims 3 to 14, **characterized in that** the concentration of NADH in the mixture obtained in step a) is 20 µM to 250 µM.

16. The method as claimed in any one of claims 3 to 15, **characterized in that** said catalytic enzyme E_{C} is chosen from the group consisting of aspartate aminotransferase (AST), alanine aminotransferase (ALT), pyruvate kinase, creatine kinase and glycerol kinase.

17. The method as claimed in claim 16, **characterized in that** said catalytic enzyme E_{C} is chosen from the group consisting of AST and ALT.

18. The method as claimed in any one of claims 3 to 17, **characterized in that** said catalytic enzyme E_{C} is aspartate aminotransferase, said substrate S₁ is L-aspartate, said substrate S₂ is α-ketoglutarate, and said catalytic enzymes E_{N} are chosen either from a malate dehydrogenase (MDH), or an MDH and a lactate dehydrogenase (LDH).

19. The method as claimed in any one of claims 3 to 17, **characterized in that** said catalytic enzyme E_{C} is alanine aminotransferase, said substrate S₁ is L-alanine, said substrate S₂ is α-ketoglutarate, and said catalytic enzyme E_{N} is a lactate dehydrogenase.

20. The use of a microfluidic chamber as defined in any one of claims 1 and 4 to 9, for assaying a catalytic plasma enzyme in a whole-blood sample.

21. The use as claimed in claim 20, **characterized in that** said catalytic enzyme is chosen from the group consisting of AST, ALT, pyruvate kinase, creatine kinase, glycerol kinase, LDH, MDH and IDH.

22. A kit or box for assaying a catalytic plasma enzyme (E_{C}) in a whole-blood sample, said enzyme E_{C} being a catalytic plasma enzyme for which one of the products obtained during the enzymatic reaction catalyzed by it is itself a substrate of at least one NADH-dependent catalytic enzyme (E_{N}) having NADH as cofactor, and said enzyme E_{C} having S₁ and S₂ as substrates, said substrate S₁ being L-aspartate and said substrate S₂ being α-ketoglutarate, **characterized in that** it comprises a microfluidic chamber as defined in any one of claims 1 and 4 to 9, and the following reagents: the substrates S₁ and S₂, NADH, at least one NADH-dependent catalytic enzyme (E_{N}), and optionally PLP.
